# EUROPÄISCHE PATENTSCHRIFT

(11) **EP 1 086 745 B1**
(45) Veröffentlichungstag und Bekanntmachung des Hinweises auf die Patenterteilung: **11.09.2002**
(21) Anmeldenummer: 00115182.8
(22) Anmeldetag: 13.07.2000
(51) Int. Cl.: B01J 25/00, B01J 25/02, B01J 37/00, C07C 29/149, C07D 307/08, C07D 307/20

(54) **Festbettkatalysatoren**
Fixed bed catalysts
Catalyseurs en lit fixe

(30) Priorität: 31.07.1999 DE 19936135
(43) Veröffentlichungstag der Anmeldung: 28.03.2001
(73) Patentinhaber: Degussa AG, 40474 Düsseldorf (DE)
(72) Erfinder: Ostgard, Daniel, Dr., 63801 Kleinostheim (DE); Berweiler, Monika, 63477 Maintal (DE); Bender, Barbara, 63517 Rodenbach (DE); Stein, Gernot, 63526 Erlensee (DE); Möbus, Konrad, Dr., 35114 Haina-Löhlbach (DE)

(56) Entgegenhaltungen:
- EP-A- 0 648 534
- DE-A- 3 537 247
- DE-A- 4 424 069
- DE-A- 4 446 907
- DE-A- 19 640 554
- GB-A- 1 029 502
- GB-A- 1 059 598
- US-A- 4 043 946

## Beschreibung

Die vorliegende Erfindung betrifft Festbettkatalysatoren und deren Verwendung bei der Hydrierung gesättigter und ungesättigter Ester.

Aktivierte Metallkatalysatoren sind auf dem Gebiet der chemischen Verfahrenstechnik als Raney-Katalysatoren bekannt. Sie werden, größtenteils in Pulverform, für eine Vielzahl von Hydrierungs-, Dehydrierungs-, Isomerisierungs, reduktiven Alkylierungs-, reduktiven Aminierungs- und Hydratisierungsreaktionen organischer Verbindungen verwendet. Diese pulverisierten Katalysatoren werden hergestellt aus einer Legierung eines katalytisch aktiven Metalls, das hierin auch als Katalysatormetall bezeichnet wird, mit einer weiteren Legierungskomponente, die in Alkalien löslich ist. Als Katalysatormetalle werden hauptsächlich Nickel, Kobalt, Kupfer oder Eisen verwendet. Als Legierungskomponente, die in Alkalien löslich ist, kommt im allgemeinen Aluminium zum Einsatz, es können aber auch andere Komponenten verwendet werden, insbesondere Zink und Silicium oder Gemische davon mit Aluminium.

Pulverisierte Katalysatoren haben den Nachteil, daß sie nur in einem diskontinuierlichen Verfahren verwendet werden können und nach der katalytischen Reaktion vom Reaktionsmedium durch eine kostspielige Sedimentation und/oder Filtration getrennt werden müssen. Aus diesem Grund wurde eine Vielzahl verschiedener Prozesse für die Herstellung von Formartikeln offenbart, die nach der Extraktion des Aluminiums zu aktivierten Metallfestbettkatalysatoren führen. Somit sind zum Beispiel grobe partikuläre Raney-Legierungen, d.h. Raney-Legierungen, die nur grob zermahlen wurden, erhältlich, die durch eine Behandlung mit Natronlauge aktiviert werden können. Extraktion und Aktivierung finden dann nur in einer Oberflächenschicht statt, deren Dicke durch während der Extraktion eingesetzte Bedingungen eingestellt werden kann.

Ein wesentlicher Nachteil der mit diesen vorherigen Methoden hergestellten Katalysatoren ist die schlechte mechanische Stabilität der aktivierten Außenschicht. Da nur diese Außenschicht der Katalysatoren katalytisch aktiv ist, kommt es durch Abrieb zu einer raschen Deaktivierung, und eine erneute Aktivierung von tieferliegenden Schichten der Legierung unter Verwendung von Natronlauge führt dann bestenfalls zu einer teilweisen Reaktivierung.

Es ist bekannt, daß Re-dotierte Pd (DE 25 19 817 A1) oder Re-dotierte Ru (WO 96/27436) Trägerkatalysatoren für die Produktion von Gamma-Butyrolacton (GBL), Tetrahydrofuran (THF) oder 1,4-Butandiol (BDO) durch Maleinsäure- oder Maleinsäureanhydrid-Hydrierung verwendet werden. Diese Systeme funktionieren als Pulver- oder Festbettkatalysatoren bei Drücken von 138 bar oder höher und bei Temperaturen von 250°C oder höher. In dieser Hinsicht wäre ein System, bei dem diese Reaktion unter milderen Bedingungen ablaufen würde, von großem Vorteil. Kupfer-Chromit ist ein weiteres Katalysatorsystem, das mit einigem Erfolg für die Esterhydrierung verwendet wurde (DE 39 03 029 A1).

Das Dokument EP 0 648 534 A1 beschreibt geformte, aktivierte Raney-Metallfestbettkatalysatoren (Metalyst®) und deren Herstellung. Diese Technologie umgeht die oben beschriebenen Nachteile, wie zum Beispiel die schlechte mechanische Stabilität aufgrund der Aktivierung einer Außenschicht. Zur Herstellung dieser Katalysatoren wird ein Pulvergemisch aus einer Katalysatorlegierung und einem Bindemittel verwendet, wobei die Katalysatorlegierung wenigstens ein katalytisch aktives Katalysatormetall und eine extrahierbare Legierungskomponente enthält. Die reinen Katalysatormetalle oder Gemische davon, die keine extrahierbaren Komponenten enthalten, werden als Bindemittel verwendet. Die Verwendung einer Bindemittelmenge zwischen 0,5 und 20 Gewichtsprozent in bezug auf die Katalysatorlegierung ist wichtig, um nach der Aktivierung eine ausreichende mechanische Stabilität zu erhalten. Nach dem Formen der Katalysatorlegierung und des Bindemittels mit konventionellen Formgebungshilfsmitteln und Porenbildnern werden die erhaltenen, frisch erzeugten Artikel bei Temperaturen unter 850°C kalziniert. Infolge von Sinterprozessen im fein zerteilten Bindemittel werden dadurch feste Verbindungen zwischen den einzelnen Körnchen der Katalysatorlegierung erzeugt. Diese Verbindungen sind im Gegensatz zu Katalysatorlegierungen nicht oder nur in geringem Maße extrahierbar, so daß selbst nach der Aktivierung eine mechanisch stabile Struktur erhalten wird, ohne daß die Festigkeit des geformten Artikels gefährdet wird.

Eine Aufgabe der vorliegenden Erfindung ist es daher, aktivierte Grundmetall-Festbettkatalysatoren bereitzustellen, die gesättigte und ungesättigte Ester unter milderen Bedingungen als existierende Technologien hydrieren, mit besseren Aktivitäten und Selektivitäten.

Die erfindungsgemäßen Festbettkatalysatoren sind dahingehend von Vorteil, daß Re-dotierte Metallkatalysatoren die Hydrierung von Maleinsäure oder Maleinsäureanhydrid in Gamma-Butyrolacton, Tetrahydrofuran oder 1,4-Butandiol bei einer Temperatur von 200°C und einem Druck von 80 bar durchführen können. Darüber hinaus waren diese Katalysatoren in der Lage, Fettsäureester in die entsprechenden Fettalkohole zu hydrieren, und zwar mit höheren Aktivitäten und Selektivitäten als herkömmliche Kupfer-Chromit-Katalysatoren.

Eine Aufgabe der vorliegenden Erfindung ist es, einen geformten, aktivierten Raney-Metallfestbettkatalysator bereitzustellen, wobei der genannte Katalysator durch ein Verfahren hergestellt wird, das im wesentlichen die folgenden Schritte umfaßt: Herstellen eines Gemischs aus Pulvern, die im wesentlichen aus wenigstens einer Katalysatorlegierung und wenigstens einem Bindemittel bestehen, und einem Anfeuchtemittel und bei Bedarf einem Zusatzstoff, ausgewählt aus der Gruppe bestehend aus einem Formgebungshilfsmittel, Schmiermittel, Plastifizierungsmittel, Porenbildner und Gemischen davon, wobei die genannte Katalysatorlegierung im wesentlichen aus wenigstens einem Raney-Prozeßmetall als katalytisch aktivem Katalysatormetall, einer auslaugbaren Legierungskomponente und bei Bedarf einem Promoter besteht, wobei das genannte Bindemittel im wesentlichen aus wenigstens einem reinen Raney-Prozeßmetall besteht, Homogenisieren des genannten Gemischs, Formen des genannten Gemischs, um einen geformten Katalysatorvorläufer zu erhalten, der noch immer nicht aktiviert ist, Kalzinieren des genannten geformten Katalysatorvorläufers bei einer Temperatur unter 850°C, um einen gesinterten Katalysatorvorläufer zu erzeugen, und Aktivieren des genannten gesinterten Katalysatorvorläufers durch Auslaugen der genannten auslaugbaren Legierungskomponente mit Alkali, bis die ausgelaugte und damit aktivierte Außenschicht eine einstellbare Dicke zwischen 0,05 bis 1.0 mm hat, die bis zu 70% oder mehr des aktivierten geformten Formteils entspricht, und anschließend Waschen des endgültigen Katalysators; Dotieren des genannten Katalysators mit Rhenium als Promotor nach dem genannten Aktivieren und Waschen, durch Einführen des genannten Katalysators in eine Perrheniumsäurelösung oder Lösungen aus anderen Re-Salzen über einen ausreichenden Zeitraum, um das gesamte Rhenium auf dem Katalysator zu fixieren oder durch Zugabe des Rheniums in die unaktivierte Legierung oder gegebenenfalls durch Zugabe des Rheniums in das Bindemittel. Der pH-Wert der Re-Lösung kann eingestellt werden oder nicht, und die Temperatur der Dotierungslösung kann von geringer als Raumtemperatur bis hin zu wesentlich höheren Temperaturen reichen. Ferner kann das Rhenium in die unaktivierte Legierung, das Bindemittel, gegeben werden, oder auf irgendeine andere Weise eingeführt werden, die seine Anwesenheit im Katalysator ermöglicht. Der Re-Gehalt kann zwischen 0,01% Re und 30% Re bevorzugt zwischen 0,01 und 15% Re insbesondere zwischen 0,01 und 10% Re liegen.

Die erfindungsgemäßen Katalysatoren können ein Raney-Metall, zum Beispiel Nickel, enthalten, das zusätzlich mit 0,01 bis 5 % Chrom und/oder mit 0,01 bis 5 % Eisen vor oder nach der Re-Dotierung dotiert wird.

Das Raney-Metall kann zusätzlich mit anderen Promotoren wie Ti, Zr, V, Ta, Mo, W, Ru, Pd, Pt, Cu, Zn oder Co vor oder nach der Re-Dotierung dotiert werden.

Bevorzugte Raney-Prozeßmetalle sind Nickel, Kobalt, Kupfer oder Kombinationen davon, und die zum Einsatz kommenden auslaugbaren Legierungskomponenten sind Aluminium, Zink, Silicium oder Kombinationen davon. Diese werden im allgemeinen in Alkali wie NaOH ausgelaugt. Das Gewichtsverhältnis zwischen Raney-Prozeßmetall und auslaugbarer Legierungskomponente in der Katalysatorlegierung liegt zwischen 10:90 und 90:10, wie es bei Raney-Legierungen üblich ist. Das als Bindemittel verwendete Raney-Prozeßmetall braucht in einer wirklichen, praktischen Anwendung nicht dem in der Katalysatorlegierung anwesenden Katalysatormetall zu entsprechen. Statt dessen besteht die Möglichkeit, verschiedene Raney-Prozeßmetalle miteinander sowie mit Promotormetallen in der Katalysatorlegierung und als Bindemittel zu kombinieren, wodurch sich ein weiteres wichtiges Maß an Freiheit bei der Einstellung der katalytischen Eigenschaften für den speziellen katalytischen Prozeß ergibt. Das in der vorliegenden Erfindung verwendete Bindemittel kann somit Nickel, Kobalt, Kupfer, Eisen und bei Bedarf Promotormetalle umfassen. Im allgemeinen sind alle Metalle geeignet, die zur Herstellung von Raney-Metallkatalysatoren verwendet werden. Das Bindemittelmetall wird in einer nicht auslaugbaren und unverfälschten Form verwendet.

Katalysatorlegierung und Bindemittel werden in Pulverform verarbeitet, typischerweise unter Zugabe von Anfeuchtemitteln und bei Bedarf unter Zugabe von konventionellen Zusatzstoffen wie Formgebungshilfsmittel, Schmiermittel, Plastifizierungsmittel und bei Bedarf Porenbildner, um ein formbares Material zu erhalten. Jedes Material, das konventionell für diese Zwecke verwendet wird, kann als Formgebungshilfsmittel, Schmiermittel, Plastifizierungsmittel und Porenbildner verwendet werden. Eine Reihe für diesen Zweck geeigneter Materialien wird in den folgenden US-Patenten erwähnt (die hiermit alle durch Bezugnahme in ihrer Gesamtheit eingeschlossen sind): 4,826,799; 3,404,551 und 3,351,495. Wachse wie zum Beispiel Wachs C Mikropulver PM von Hoechst AG, Fette wie Magnesiumoder Aluminiumstearate oder Polymere, die Kohlenhydrate wie Tylose (Methylcellulose) enthalten, werden für die obigen Zwecke bevorzugt verwendet.

Die Feststoffe in dem Gemisch werden in geeigneten konventionellen Mischern oder Knetern unter Zugabe eines Anfeuchtemittels sorgfältig homogenisiert. Wasser, Alkohole, Glykole, Polyetherglykole oder Gemische davon sind als Anfeuchtemittel geeignet, wie in Fachkreisen gut bekannt ist.

Der Primärpartikelgrößenbereich der verwendeten Pulver von Katalysatorlegierung und Bindemittel bleibt während der Homogenisierung im wesentlichen unverändert. Das heißt, es findet kein Zermahlen statt.

Zweck dieser Vorbehandlung mit dem Anfeuchtemittel und den Zusatzstoffen ist es, das Gemisch für den nachfolgenden Formungsprozeß vorzubereiten. Extrusion, Pelletierung und Kompression können zum Beispiel für den Formgebungsprozeß unter Verwendung konventioneller Ausrüstung, die für solche Zwecke bekannt ist, durchgeführt werden.

Art und Reihenfolge des Einbaus von Zusatzstoffen sind von dem angewendeten Formgebungsprozeß abhängig. Extrusion setzt ein Kunststoffmaterial mit einer spezifischen Viskosität voraus, wohingegen die Pelletierung ein freifließendes Material verlangt, das problemlos dosiert werden kann. Die für diesen Zweck anzuwendenden Techniken, wie zum Beispiel Agglomeration, um ein.freifließendes Pulver herzustellen, oder Einstellen der korrekten Viskosität für die Extrusion, sind der fachkundigen Person routinemäßig bekannt. Es ist nur wichtig, daß der Primärpartikelgrößenbereich des Katalysatorpulvers und Bindemittelpulvers durch die Vorbehandlung im wesentlichen unverändert bleibt.

Alle auf dem Gebiet der Katalysatoren üblichen Formen sind für die Formartikel geeignet. Je nach den Anforderungen der speziellen Anwendung können Sphären, Ringe, Speichenringe oder Pellets hergestellt werden.

Die endgültigen Formartikel werden bedarfsabhängig auf Gewichtskonstanz bei Temperaturen zwischen 80°C und 120°C getrocknet und dann bei Temperaturen unter 850°C, vorzugsweise zwischen 500°C und 700°C, in Luft in kontinuierlich oder diskontinuierlich betriebenen Kalzinieröfen wie Drehöfen oder stationären Öfen kalziniert. Die organischen Zusatzstoffe brennen dann ab und lassen ein entsprechendes Porensystem zurück.

Die Porenstruktur und das Porenvolumen der Katalysatoren kann durch die Wahl geeigneter porenbildender Zusatzstoffe über einen großen Bereich variiert werden. Die endgültig entwickelte Porenstruktur und das Porenvolumen werden auch von der Partikelgröße der Pulver der verwendeten Katalysatorlegierung und des verwendeten Bindemittels beeinflußt.

Die Struktur des Formartikels kann durch die Wahl der geeigneten erwähnten Parameter den Anforderungen an den speziellen katalytischen Prozeß angepaßt werden.

Während der Kalzinierung der Formartikel backen das Katalysatorlegierungspulver und das Bindemittelpulver zusammen und stellen Formartikel mit hoher mechanischer Stabilität und guter Abriebbeständigkeit bereit. Typischerweise liegt die Härte der zylindrischen Pellets nach der Kalzinierung zwischen 200 und 300 N (radial gemäß ASTM D 4179-82 gemessen).

Nach der Kalzinierung werden die Formartikel aktiviert, indem das Aluminium mit Natronlauge ausgelaugt wird. Für diesen Zweck kann eine 20%ige Natriumhydroxidlösung verwendet werden, die auf 80°C erwärmt wurde. In diesem Fall führt eine 2-stündige Behandlung zu einer aktiven Außenschicht mit einer Dicke von etwa 0,1 bis 1 mm. Überraschenderweise wurde nachgewiesen, daß die Härte durch Auslaugen tatsächlich leicht zunimmt, im Falle der Pellets auf Werte von mehr als 300 N.

Diese Eigenschaften sind eng mit dem reinen Raney-Prozeßmetall verbunden, das als Bindemittel verwendet wird, das während des Auslaugens nicht herausgelöst wird und somit im gesinterten Produkt stabile Bindungen zwischen den einzelnen legierten Partikeln bildet. Gemäß dem deutschen Patent DE 197 218 98.9 (Freund, Berweiler, Bender und Kempf; 1998) kann die Verwendung eines metallischen Bindemittels zur Herstellung von Metalyst® vermieden werden, wenn die Phasenbereiche der Legierung klein genug sind. Die Größe der Phasenbereiche kann mit der Kühlgeschwindigkeit und dem Kühlverfahren der Legierung gesteuert werden. Die Verwendung eines Bindemittels in der vorliegenden Erfindung ist somit fakultativ, und die Technologie von DE 197 218 98.9 ist auf den erfindungsgemäßen Katalysator anwendbar.

Die Wahl der Metalle, die als Bindemittel verwendet werden, wirkt sich möglicherweise auf die katalytische Aktivität aus. Durch Beschränken der Kalzinierungstemperatur auf Werte unter 850°C wird die Bildung von Alpha-Aluminiumoxid verhindert, wie anhand einer Röntgenkristallstrukturanalyse des kalzinierten Materials zu sehen ist. Gebildetes γ-Aluminiumoxid wird aus der Katalysatorstruktur herausgelöst, wenn der Katalysator mit Natronlauge aktiviert wird.

Das Fehlen von α-Aluminiumoxid im Katalysator wird bei der Aktivierung sehr deutlich. Während erfindungsgemäße Katalysatoren unter recht milden Bedingungen (20% NaOH, 80°C) innerhalb von nur 2 Stunden aktiviert werden können, muß die Temperatur der Alkalilösung erhöht und die Aktivierungszeit verlängert werden, wenn mit α-Aluminiumoxid gebundene Katalysatoren (gemäß US-Patent Nr. 4,826,799) aktiviert werden, um eine aktive Außenschicht der gleichen Dicke zu erhalten.

Zur Herstellung des erfindungsgemäßen Katalysators können die durchschnittliche Partikelgröße des Katalysatorlegierungspulvers und des Bindemittels und auch das Gewichtsverhältnis von Katalysatorlegierungspulver zu Bindemittel über einen großen Bereich variiert werden. Da das Bindemittel auch zur katalytischen Aktivität beiträgt, es aber durch Extrahieren von Aluminium nicht aktiviert werden kann, ist sein möglicher Beitrag zur katalytischen Aktivität begrenzt. Sein Anteil im Katalysator sollte daher möglichst gering gehalten werden.

Gewichtsverhältnisse zwischen Katalysatorlegierungspulver und Bindemittel im Bereich von 100:20 bis 100:0,5 haben sich als nützlich erwiesen. Es wurde auch gezeigt, daß die Partikelgröße des Bindemittels geringer sein sollte als die Partikelgröße des Katalysatorlegierungspulvers. Partikel des Bindemittels können dann als kleine Brücken zwischen den größeren legierten Partikeln angesehen werden. Es wurde gefunden, daß die Härte der endgültigen Katalysatorstruktur mit abnehmender Partikelgröße des Bindemittels innerhalb gewisser Grenzen zunahm. Angemessene Aktivitätswerte werden erhalten, wenn die durchschnittliche Partikelgröße des Katalysatorlegierungspulvers zwischen 10 und 500 µm liegt.

Beim Dotieren mit Rhenium ist es ratsam, die Dotierung erst nach der Aktivierung des Katalysators durchzuführen. Dazu wird der endgültige Katalysator in eine Rheniumlösung (z.B. Perrheniumsäure) gegeben. Die Menge an Rhenium und die für seine Zugabe benötigte Zeit können durch Einstellen des pH-Wertes und der Temperatur der Rheniumlösung geregelt werden. Je nach Behandlungsart wird eine spezifische Menge der Rheniumverbindung vom Katalysator adsorbiert, z.B. bis zu 20 Gew.-%.

Eine weitere Aufgabe der Erfindung ist es, ein Verfahren zur Herstellung des geformten Raney-Metallfestbettkatalysators bereitzustellen, wobei das genannte Verfahren im wesentlichen die folgenden Schritte umfaßt: Herstellen eines Gemischs aus Pulvern, die im wesentlichen aus wenigstens einer Katalysatorlegierung und bei Bedarf einem Bindemittel bestehen, und einem Anfeuchtemittel und bei Bedarf einem Zusatzstoff, ausgewählt aus der Gruppe bestehend aus einem Formgebungshilfsmittel, Schmiermittel, Plastifizierungsmittel, Porenbildner und Gemischen davon, wobei die genannte Katalysatorlegierung im wesentlichen aus wenigstens einem Raney-Prozeßmetall als katalytisch aktive Komponente, einer auslaugbaren Legierungskomponente und bei Bedarf einem Promotor besteht, wobei das genannte fakultative Bindemittel im wesentlichen aus wenigstens einem Raney-Prozeßmetall besteht, Homogenisieren des genannten Gemischs, Formen des genannten Gemischs, um einen geformten Katalysatorvorläufer zu erhalten, der noch immer nicht aktiviert ist, Kalzinieren des genannten geformten Katalysatorvorläufers bei einer Temperatur unter 850°C, um einen gesinterten Katalysatorvorläufer zu erzeugen, und Aktivieren des genannten gesinterten Katalysatorvorläufers durch Auslaugen der genannten auslaugbaren Legierungskomponente mit Alkali, bis die ausgelaugte und damit aktivierte Außenschicht eine Dicke zwischen 0,05 und 1 mm oder mehr hat; anschließend bei Bedarf Waschen des endgültigen Katalysators; Dotieren mit Rhenium als Promotor nach dem genannten Aktivieren und Waschen, durch Einführen des genannten Katalysators in eine Rheniumlösung, wobei die Eigenschaften der Rheniumablagerung durch die Temperatur und den pH-Wert der Rheniumlösung geregelt werden können.

Eine weitere Aufgabe der vorliegenden Erfindung ist die Verwendung von Rhenium-dotiertem Raney-Metallfestbettkatalysator für die Hydrierung ungesättigter und gesättigter Ester. Ein Beispiel für diese Technologie ist die Hydrierung von Maleinsäureanhydrid in γ-Butyrolacton, Tetrahydrofuran, 1,4-Butandiol unter milden Bedingungen. Ferner wurde gefunden, daß die vorliegende Erfindung ein exzellenter Katalysator für die Hydrierung von Fettsäureester in Fettalkohol ist.

### 1. Vergleichsbeispiel

Ein im Handel erhältlicher 2% Pd-Katalysator auf 4 mm Kohlenstoffextrudaten wird mit 0,5% Re dotiert, indem er in einer Perrheniumsäurelösung behandelt wird, deren pH-Wert mit Natriumhydroxid auf 10,5 eingestellt wird, bevor der Katalysator in die Lösung gegeben wird. Die Behandlung wird so lange fortgesetzt, bis kein Rhenium mehr in der Rheniumlösung vorhanden ist. Der Einsatz dieses Katalysators gemäß Anwendungsbeispiel 1 für eine Maleinsäureanhydridhydrierung erbrachte nach einer Reaktionszeit von 5 Stunden 10,5% Gamma-Butyrolacton, 0,0% Tetrahydrofuran und 0,0% 1,4-Butandiol.

### 1. Beispiel

Ein freifließendes, pelletierbares Katalysatorgemisch wird gemäß den Anweisungen in der EP 0 648 534 A1 für einen Katalysator aus 1000 g 50% Ni und 50% Al Legierungspulver, 7,5 g reinem Nickelpulver (99% Ni und d50 = 21 µm) und 50 g Ethylen-Bis-Stearoylamid hergestellt. Aus diesem Gemisch werden Tabletten mit einem Durchmesser von 4 mm und einer Dicke von 4 mm komprimiert. Die geformten Artikel werden 2 Stunden lang bei 700°C kalziniert. Nach der Kalzinierung werden die Tabletten 2 Stunden lang bei 80°C in 20%iger Natronlauge aktiviert. Dieser Katalysator wird auf 0,45% Re dotiert, indem er 48 Stunden lang einer gerührten Perrheniumsäurelösung ausgesetzt wird, deren pH-Wert auf 10,5 eingestellt wird, bevor der Katalysator zugegeben wird. Der Einsatz dieses Katalysators gemäß Anwendungsbeispiel 1 für eine Maleinsäureanhydridhydrierung erbrachte nach einer Reaktionszeit von 5 Stunden 71,6% Gamma-Butyrolacton, 4,7% Tetrahydrofuran und 1,3% 1,4-Butandiol.

### 2. Vergleichsbeispiel

Im Handel erhältliche Kupfer-Chromit-Extrudate von Mallinckrodt wurden als Vergleichstechnologie für die Hydrierung von Fettsäureestern verwendet. Bei Einsatz gemäß Anwendungsbeispiel 2 erbrachte dieser Katalysator nach einer Reaktionszeit von 300 Minuten bei 230°C 20,78% Fettalkohol mit einer Verseifungszahl von 136,6. Die Farbe des Reaktanten war zu diesem Zeitpunkt bläulich-grün, was auf die Anwesenheit gelöster Metalle schließen ließ. Bei einem Einsatz gemäß Anwendungsbeispiel 3 erbrachte dieser Katalysator nach einer Reaktionszeit von 1230 Minuten bei 200°C 20,5% Fettalkohol mit einer Verseifungszahl von 143,5.

### 2. Beispiel

Ein freifließendes, pelletierbares Katalysatorgemisch wird gemäß den Anweisungen in der EP 0 648 534 A1 für einen Katalysator aus 1000 g 50% Ni und 50% Al Legierungspulver, 7,5 g reinem Nickelpulver (99% Ni und d50 = 21 µm) und 50 g Ethylen-Bis-Stearoylamid hergestellt. Aus diesem Gemisch werden Tabletten mit einem Durchmesser von 4 mm und einer Dicke von 4 mm komprimiert. Die geformten Artikel werden 2 Stunden lang bei 700°C kalziniert. Nach der Kalzinierung werden die Tabletten 2 Stunden lang bei 80°C in 20%iger Natronlauge aktiviert. Dieser Katalysator wird auf 2,0% Re dotiert, indem er 100 Stunden lang einer gerührten Perrheniumsäurelösung ausgesetzt wird, deren pH-Wert auf 10,5 eingestellt wird, bevor der Katalysator zugegeben wird. Der Einsatz dieses Katalysators gemäß Anwendungsbeispiel 1 für eine Maleinsäureanhydridhydrierung erbrachte nach einer Reaktionszeit von 5 Stunden 55% Gamma-Butyrolacton, 14,2% Tetrahydrofuran und 1,3% 1,4-Butandiol.

### 3. Beispiel

Ein freifließendes, pelletierbares Katalysatorgemisch wird gemäß den Anweisungen in der EP 0 648 534 A1 für einen Katalysator aus 1000 g 50% Ni und 50% Al Legierungspulver, 7,5 g reinem Nickelpulver 99% Ni und d50 = 21 µm) und 50 g Ethylen-Bis-Stearoylamid hergestellt. Aus diesem Gemisch werden Tabletten mit einem Durchmesser von 4 mm und einer Dicke von 4 mm komprimiert. Die geformten Artikel werden 2 Stunden lang bei 700°C kalziniert. Nach der Kalzinierung werden die Tabletten 2 Stunden lang bei 80°C in 20%iger Natronlauge aktiviert. Dieser Katalysator wird auf 4,0%. Re dotiert, indem er 192 Stunden lang einer gerührten Perrheniumsäurelösung ausgesetzt wird, deren pH-Wert auf 10,5 eingestellt wird, bevor der Katalysator zugegeben wird. Der Einsatz dieses Katalysators gemäß Anwendungsbeispiel 1 für eine Maleinsäureanhydridhydrierung erbrachte nach einer Reaktionszeit von 5 Stunden 40% Gamma-Butyrolacton, 3,5% Tetrahydrofuran und 26,25% 1,4-Butandiol.

### 4. Beispiel

Ein freifließendes, pelletierbares Katalysatorgemisch wird gemäß den Anweisungen in der EP 0 648 534 A1 für einen Katalysator aus 1000 g 50% Ni und 50% Al Legierungspulver, 7,5 g reinem Nickelpulver (99% Ni und d50 = 21 µm) und 50 g Ethylen-Bis-Stearoylamid hergestellt. Aus diesem Gemisch werden Tabletten mit einem Durchmesser von 4 mm und einer Dicke von 4 mm komprimiert. Die geformten Artikel werden 2 Stunden lang bei 700°C kalziniert. Nach der Kalzinierung werden die Tabletten 2 Stunden lang bei 80°C in 20%iger Natronlauge aktiviert. Dieser Katalysator wird auf 6,0% Re dotiert, indem er 240 Stunden lang einer gerührten Perrheniumsäurelösung ausgesetzt wird, deren pH-Wert auf 10,5 eingestellt wird, bevor der Katalysator zugegeben wird. Der Einsatz dieses Katalysators gemäß dem Anwendungsbeispiel für Maleinsäureanhydridhydrierung erbrachte nach einer Reaktionszeit von 5 Stunden 25,9% Gamma-Butyrolacton, 3,71% Tetrahydrofuran und 26,9% 1,4-Butandiol.

### 5. Beispiel

Ein freifließendes, pelletierbares Katalysatorgemisch wird gemäß den Anweisungen in der EP 0 648 534 A1 für einen Katalysator aus 1000 g 50% Ni und 50% Al Legierungspulver, 430 g reinem Nickelpulver (d50 = 23 µm) und 50 g Ethylen-Bis-Stearoylamid hergestellt. Aus diesem Gemisch werden Tabletten mit einem Durchmesser von 3 mm und einer Dicke von 3 mm komprimiert. Die geformten Artikel werden 6 Stunden lang bei 700°C kalziniert. Nach der Kalzinierung werden die Tabletten 2 Stunden lang bei 80°C in 20%iger Natronlauge aktiviert. Bei einem Einsatz gemäß Anwendungsbeispiel 2 erbrachte dieser Katalysator nach einer Reaktionszeit von 300 Minuten bei 230°C 3,32% Fettalkohol mit einer Verseifungszahl von 176,0. Die Farbe des Reaktanten war zu dieser Zeit weiß, was auf die Abwesenheit gelöster Metalle hinwies. Zwar erbringt dieser Katalysator bereits eine ähnliche (wenn nicht geringfügig bessere) Leistung als der aktivierte Nickelkatalysator, so kann seine Aktivität durch die Verwendung von weniger Bindemittel und eine Verringerung der Kalzinierungsheftigkeit verbessert werden. Eine Re-Dotierung könnte dieses Katalysatorsystem daher wenigstens im gleichen Maße wie der aktivierte Ni-Katalysator verbessern.

### 3. Vergleichsbeispiel

Ein freifließendes, pelletierbares Katalysatorgemisch wird gemäß den Anweisungen in der EP 0 648 534 A1 für einen Katalysator aus 1000 g 50% Ni und 50% Al Legierungspulver, 7,5 g reinem Nickelpulver (99% Ni und d50 = 21 µm) und 50 g Ethylen-Bis-Stearoylamid hergestellt. Aus diesem Gemisch werden Tabletten mit einem Durchmesser von 3 mm und einer Dicke von 3 mm komprimiert. Die geformten Artikel werden 2 Stunden lang bei 700°C kalziniert. Nach der Kalzinierung werden die Tabletten 2 Stunden lang bei 80°C in 20%iger Natronlauge aktiviert. Bei einem Einsatz gemäß Anwendungsbeispiel 2 erbrachte dieser Katalysator nach einer Reaktionszeit von 300 Minuten bei 230°C 1,92% Fettalkohol mit einer Verseifungszahl von 174,2. Die Farbe des Reaktanten war zu diesem Zeitpunkt weiß, was auf die Abwesenheit gelöster Metalle hinwies.

### 6. Beispiel

Ein freifließendes, pelletierbares Katalysatorgemisch wird gemäß den Anweisungen in der EP 0 648 534 A1 für einen Katalysator aus 1000 g 50% Ni und 50% Al Legierungspulver, 7,5 g reinem Nickelpulver (99% Ni und d50 = 21 µm) und 50 g Ethylen-Bis-Stearoylamid hergestellt. Aus diesem Gemisch werden Tabletten mit einem Durchmesser von 3 mm und einer Dicke von 3 mm komprimiert. Die geformten Artikel werden 2 Stunden lang bei 700°C kalziniert. Nach der Kalzinierung werden die Tabletten 2 Stunden lang bei 80°C in 20%iger Natronlauge aktiviert. Dieser Katalysator wird auf 3,0% Re dotiert, indem er 72 Stunden lang einer gerührten Perrheniumsäurelösung ausgesetzt wird, die auf einen pH-Wert von 10,5 eingestellt wird, bevor der Katalysator zugegeben wird. Bei einem Einsatz gemäß Anwendungsbeispiel 2 erbrachte dieser Katalysator nach einer Reaktionszeit von 300 Minuten bei 230°C 25,39% Fettalkohol mit einer Verseifungszahl von 107,9. Die Farbe des Reaktanten war zu diesem Zeitpunkt weiß, was auf die Abwesenheit gelöster Metalle hinwies. Bei einem Einsatz gemäß Anwendungsbeispiel 3 erbrachte dieser Katalysator nach einer Reaktionszeit von 1230 Minuten bei 200°C 63,85% Fettalkohol mit einer Verseifungszahl von 61,2.

### 4. Vergleichsbeispiel

Ein freifließendes, pelletierbares Katalysatorgemisch wird gemäß den Anweisungen in der EP 0 648 534 A1 für einen Katalysator aus 1000 g 40,0% Ni, 58,5% Al, 1,0% Cr und 0,5% Fe Legierungspulver, 7,5 g reinem Nickelpulver (99% Ni und d50 = 21 µm) und 50 g Ethylen-Bis-Stearoylamid hergestellt. Aus diesem Gemisch werden Tabletten mit einem Durchmesser von 3 mm und einer Dicke von 3 mm komprimiert. Die geformten Artikel werden 2 Stunden lang bei 700°C kalziniert. Nach der Kalzinierung werden die Tabletten 2 Stunden lang bei 80°C in 20%iger Natronlauge aktiviert. Bei einem Einsatz gemäß Anwendungsbeispiel 2 erbrachte dieser Katalysator nach einer Reaktionszeit von 300 Minuten bei 230°C 6,72% Fettalkohol mit einer Verseifungszahl von 160,7. Die Farbe des Reaktanten war zu diesem Zeitpunkt weiß, was auf die Abwesenheit gelöster Metalle hinwies.

### 7. Beispiel

Ein freifließendes, pelletierbares Katalysatorgemisch wird gemäß den Anweisungen in der EP 0 648 534 A1 für einen Katalysator aus 1000 g 40,0% Ni, 58,5% Al, 1,0% Cr und 0,5 % Fe Legierungspulver, 7,5 g reinem Nickelpulver (99% Ni und d50 = 21 µm) und 50 g Ethylen-Bis-Stearoylamid hergestellt. Aus diesem Gemisch werden Tabletten mit einem Durchmesser von 3 mm und einer Dicke von 3 mm komprimiert. Die geformten Artikel werden 2 Stunden lang bei 700°C kalziniert. Nach der Kalzinierung werden die Tabletten 2 Stunden. lang bei 80°C in 20%iger Natronlauge aktiviert. Dieser Katalysator wird auf 3,0% Re dotiert, indem er 72 Stunden lang einer gerührten Perrheniumsäurelösung ausgesetzt wird, die auf einen pH-Wert von 10,5 eingestellt wird, bevor der Katalysator zugegeben wird. Bei einem Einsatz gemäß Anwendungsbeispiel 2 erbrachte dieser Katalysator nach einer Reaktionszeit von 300 Minuten bei 230°C 34,2% Fettalkohol mit einer Verseifungszahl von 98,8. Die Farbe des Reaktanten war zu diesem Zeitpunkt weiß, was auf die Abwesenheit gelöster Metalle hinwies.

### Anwendungsbeispiel 1

Die katalytische Aktivität der Katalysatoren aus dem 1. Vergleichsbeispiel und den Beispielen 1 bis 4 wird anhand ihrer Fähigkeit zur Hydrierung von Maleinsäureanhydrid verglichen. Zu diesem Zweck werden 7 g Maleinsäureanhydrid und 250 g 1,4-Dioxan in einen Rührautoklaven mit einer Kapazität von 0,5 l gegeben, der mit einem mit 1000 UPM schleudernden Gasrührer ausgestattet ist. Die Gramm-Menge des Katalysators wird jedesmal in dem Rührautoklaven mit einem Katalysatorkorb aufgehängt, so daß das Katalysatormaterial mit dem Reaktanten/Lösungsmittelgemisch gründlich gewaschen wird, bevor Wasserstoff zugegeben wird. Diese Hydrierung wird bei einem Wasserstoffdruck von 80 bar und einer Temperatur von 200°C durchgeführt, wobei die Reaktion nach 5 Stunden unterbrochen wird. Proben werden nach 2 und 5 Stunden entnommen und per Gaschromatographie analysiert, um die Katalysatoraktivität und -selektivität zu bestimmen. Die Ergebnisse dieser Tests sind in Tabelle 3 enthalten.

### Anwendungsbeispiel 2

Die katalytische Aktivität der Katalysatoren aus den Vergleichsbeispielen 2-4 und Beispielen 5-7 wird anhand ihrer Fähigkeit zur Hydrierung eines Gemischs aus gesättigten und ungesättigten C-16 und C18 Estern in die Fettalkohole verglichen. Der Reaktant war ein handelsüblicher Einsatzstoff aus etwa 70% C-18 und 30% C-16 Methylester; wobei etwa 60% des gesamten Einsatzstoffes ungesättigt waren. Der Einsatzstoff enthielt auch eine sehr geringe Menge an C-12, C-14 und C-20 Methylester, wobei der Gesamtbetrag dieser anderen Ester weniger als 1% betrug. Die Hydrierung von 500 ml des obengenannten Fettsäureester-Einsatzstoffes wird mit 72 g Katalysator bei 200 bar und 230°C in einem 1L-Rührautoklaven durchgeführt, der mit einem Blasenrührer und einem Katalysatorkorb ausgestattet ist. Nach einer Reaktionszeit von 300 Minuten wird eine Probe entnommen, die per Gaschromatographie analysiert und deren Verseifungszahl bestimmt wird. Die Verseifungszahl wird durch Mischen von 2,0 g (± 0,1 mg) der Hydrierungsprobe mit 50 ml einer ethanolischen 0,5 N KOH Lösung und 60-minütiges Erhitzen unter Rückfluß auf den Siedepunkt der.Lösung bestimmt. Anschließend wird eine 1,0%ige Phenolphthaleinlösung in die warme Lösung gegeben und mit einer 0,5 N HCl-Standardlösung titriert, um die restliche KOH-Menge zu bestimmen. Ein Blindversuch ohne Hydrierungsprobe wird zur gleichen Zeit unter den gleichen Bedingungen als Vergleich durchgeführt. Die Verseifungszahl entspricht der mg-Menge von KOH, die notwendig ist, um ein Gramm des Fettsäureestergemischs zu verseifen, wobei eine höhere Zahl darauf hinweist, daß das Gemisch eine höhere Prozentzahl Ester enthält. In den hierin benutzten Vergleichen bedeutet eine höhere Verseifungszahl, daß während der Hydrierung eine geringere Menge des Fettsäureesters in den entsprechenden Fettalkohol umgewandelt wurde. Die Daten für diese Experimente sind in Tabelle 1 aufgeführt.

### Anwendungsbeispiel 3

Die katalytische Aktivität der Katalysatoren aus dem 2. Vergleichsbeispiel und dem 6. Beispiel wird ebenfalls anhand ihrer Fähigkeit zur Hydrierung des oben im 2. Anwendungsbeispiel erwähnten Gemischs aus gesättigten und ungesättigten C-16 und C-18 Ester in Alkohole verglichen, und zwar unter den gleichen Bedingungen, mit der Ausnahme, daß die Reaktionstemperatur 200°C beträgt. Proben werden nach 300 und 1230 Minuten entnommen, um eine Analyse per Gaschromatographie durchzuführen und die Verseifungszahl zu bestimmen (Beschreibung siehe oben). Die Daten dieser Experimente sind in Tabelle 2 aufgeführt.

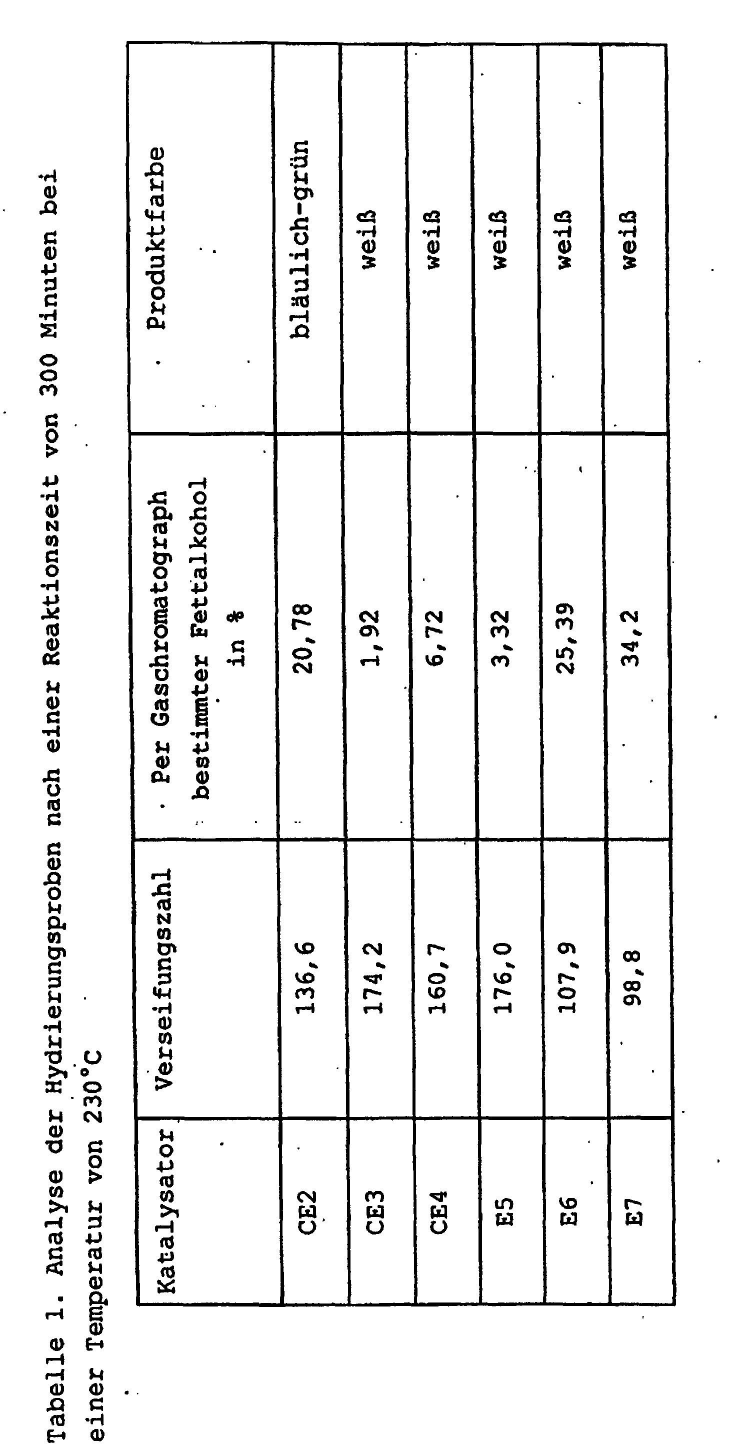

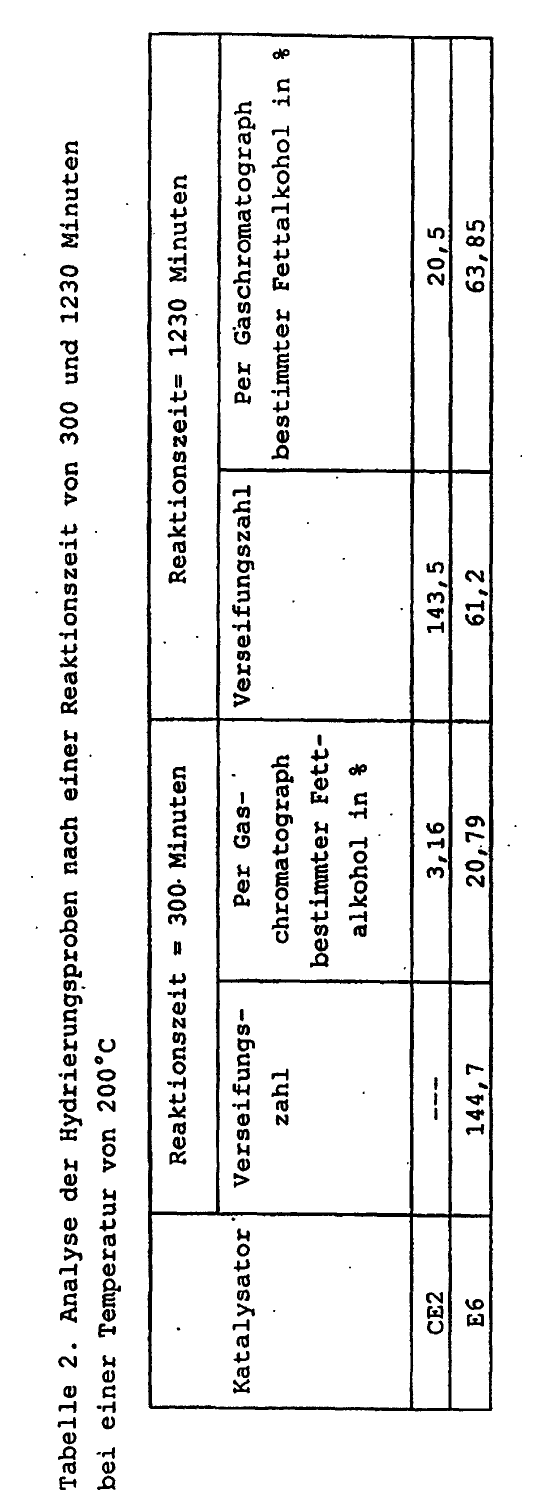

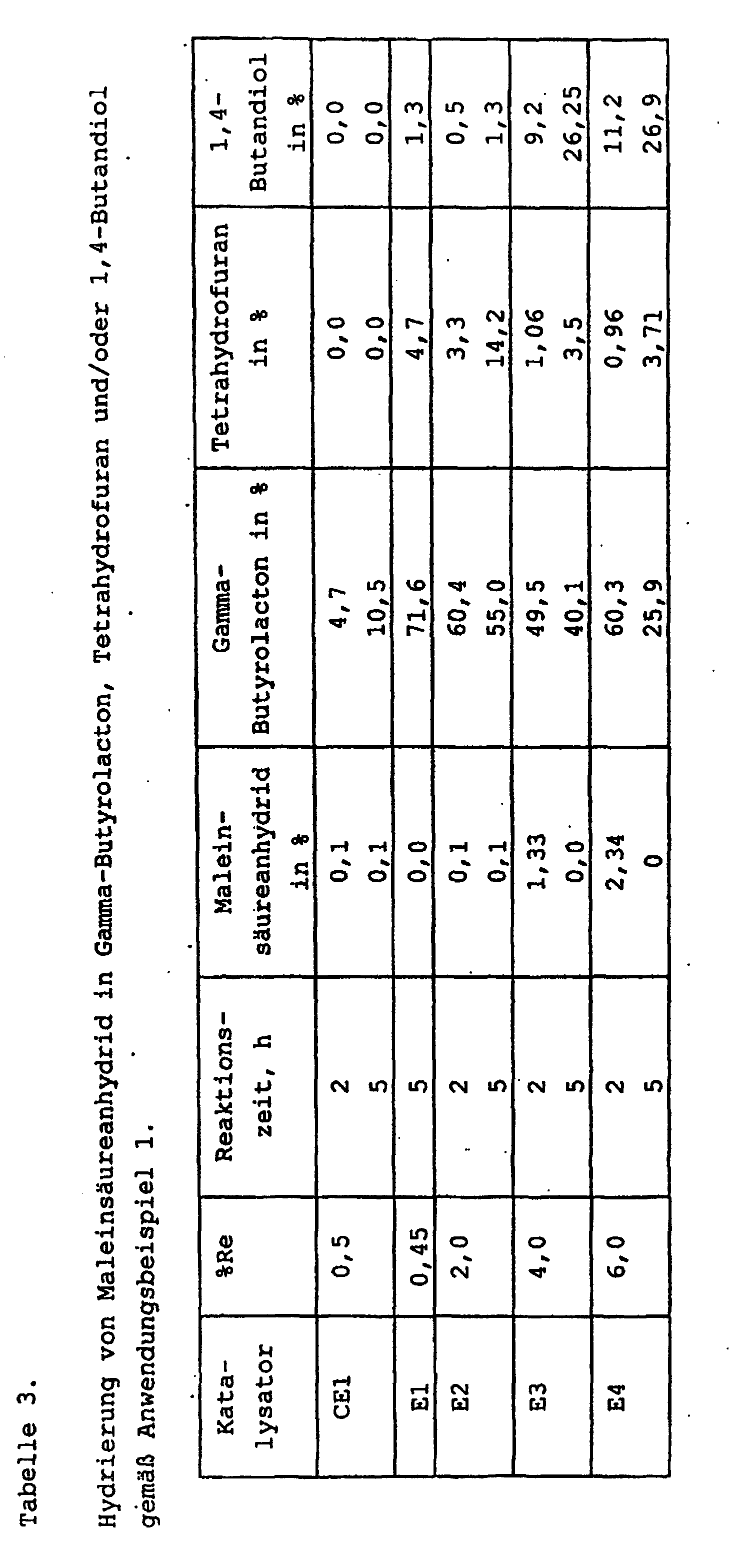

## Patentansprüche

1. Geformter, aktivierter Raney-Metallfestbettkatalysator, erhältlich Katalysator durch ein Verfahren, das im wesentlichen die folgenden Schritte umfaßt: Herstellen eines Gemischs aus Pulvern, die im wesentlichen aus wenigstens einer Katalysatorlegierung und gegebenenfalls wenigstens einem Bindemittel bestehen, und einem Anfeuchtemittel und bei Bedarf einem Zusatzstoff, ausgewählt aus der Gruppe bestehend aus einem Formgebungshilfsmittel, Schmiermittel, Plastifizierungsmittel, Porenbildner und Gemischen davon, wobei die genannte Katalysatorlegierung im wesentlichen aus wenigstens einem Raney-Prozeßmetall als katalytisch aktives Katalysatormetall, einer auslaugbaren Legierungskomponente und bei Bedarf einem Promotor besteht, wobei das genannte Bindemittel im wesentlichen aus wenigstens einem reinen Raney-Prozeßmetall besteht, Homogenisieren des.genannten Gemischs, Formen des genannten Gemischs, um einen geformten Katalysatorvorläufer zu erhalten, der noch immer nicht aktiviert ist, Kalzinieren des genannten geformten Katalysatorvorläufers bei einer Temperatur unter 850°C, um einen gesinterten Katalysatorvorläufer zu erzeugen, und Aktivieren des genannten gesinterten Katalysatorvorläufers durch Auslaugen der genannten auslaugbaren Legierungskomponente mit Alkali, bis die ausgelaugte und damit aktivierte Außenschicht eine einstellbare Dicke zwischen 0,05 und 1 mm hat, und anschließend Waschen des endgültigen Katalysators; Dotieren des genannten Katalysators mit Rhenium als Promotor nach dem genannten Aktivieren, durch Einführen des genannten Katalysators in eine Perrheniumsäurelösung oder Lösungen aus anderen Re-Salzen über einen ausreichenden Zeitraum, um das gesamte Rhenium auf dem Katalysator zu fixieren oder durch Zugabe des Rheniums in die unaktivierte Legierung oder gegebenenfalls durch Zugabe des Rheniums in das Bindemittel.

2. Verfahren zum Herstellen des geformten Raney-Metallfestbettkatalysators nach Anspruch 1, wobei das genannte Verfahren im wesentlichen die folgenden Schritte umfaßt: Herstellen eines Gemischs aus Pulvern, die im wesentlichen aus wenigstens einer Katalysatorlegierung und gegebenenfalls wenigstens einem Bindemittel bestehen, und einem Anfeuchtemittel und bei Bedarf einem Zusatzstoff, ausgewählt aus der Gruppe bestehend aus einem Formgebungshilfsmittel, Schmiermittel, Plastifizierungsmittel, Porenbildner und Gemischen davon, wobei die genannte Katalysatorlegierung im wesentlichen aus wenigstens einem Raney-Prozeßmetall als katalytisch aktive Komponente, einer auslaugbaren Legierungskomponente und bei Bedarf einem Promotor besteht, wobei das genannte Bindemittel im wesentlichen aus wenigstens einem Raney-Prozeßmetall besteht, Homogenisieren des genannten Gemischs, Formen des genannten Gemischs, um einen geformten Katalysatorvorläufer zu erhalten, der noch immer nicht .aktiviert ist, Kalzinieren des genannten geformten Katalysatorvorläufers bei einer Temperatur unter 850°C, um einen gesinterten Katalysatorvorläufer zu erzeugen, und Aktivieren des genannten gesinterten Katalysatorvorläufers durch Auslaugen der genannten auslaugbaren Legierungskomponente mit Alkali, bis die ausgelaugte und damit aktivierte Außenschicht eine einstellbare Dicke zwischen 0,05 und 1 mm hat; anschließend bei Bedarf Waschen des endgültigen Katalysators; **dadurch gekennzeichnet, daß** der Katalysator mit Rhenium als Promotor nach dem genannten Aktivieren dotiert wird, indem der genannte Katalysator in eine Perrheniumsäurelösung eingeführt wird, deren pH-Wert zuerst über einen ausreichenden Zeitraum eingestellt wurde, um das gesamte Rhenium auf dem Katalysator zu fixieren oder das Rhenium in die unaktivierte Legierung oder gegebenenfalls durch Zugabe des Rheniums in das Bindemittel.

3. Katalysatoren nach Ansprüch 1, bei denen kein Bindemittel für einen stabilen Formkörper erforderlich ist und der resultierende Katalysator mit Re dotiert wird, indem der aktivierte Katalysator in eine Perrheniumsäurelösung über einen ausreichenden Zeitraum eingeführt wird.

4. Katalysatoren nach den Ansprüchen 1 oder 3, bei denen der Re-Gehalt zwischen 0,01% Re und 30% Re liegt.

5. Katalysatoren nach den Ansprüchen 1 oder 3, bei denen der Re-Gehalt zwischen 0,01% Re und 10% Re liegt.

6. Katalysatoren nach den Ansprüchen 1 oder 3, bei denen das Raney-Metall Ni ist.

7. Katalysatoren nach den Ansprüchen 1 oder 3, bei denen das Raney-Metall Cu ist.

8. Katalysatoren nach den Ansprüchen 1 oder 3, bei denen das Raney-Metall zusätzlich mit 0,01-5% Chrom in . Kombination mit 0,01-5% Eisen vor oder nach der Re-Dotierung wird.

9. Katalysatoren nach Ansprüche 8, bei denen das Raney-Metall Nickel ist und zusätzlich mit 0,01-5% Chrom in Kombination mit 0,01-5% Eisen vor oder nach der Re-Dotierung dotiert wird.

10. Katalysatoren nach den Ansprüchen 1 oder 3, bei denen das Raney-Metall zusätzlich mit 0,01-5% Chrom vor oder nach der Re-Dotierung dotiert wird.

11. Katalysatoren nach den Ansprüchen 1 oder 3, bei denen das Raney-Metall zusätzlich mit 0,01-5% Eisen vor oder nach der Re-Dotierung wird.

12. Katalysatoren nach den Ansprüchen 1 oder 3, bei denen das Raney-Metall zusätzlich mit anderen Promotoren wie Ti, Zr, V, Ta, Mo, W, Ru, Pd, Pt, Cu, Zn, oder Co vor oder nach der Re-Dotierung dotiert wird.

13. Katalysatoren nach den Ansprüchen 1 oder 3, bei denen die Ablagerung von Re mit dem pH-Wert der Dotierungslösung gesteuert wird.

14. Katalysatoren nach den Ansprüchen 1 oder 3, bei denen die Ablagerung von Re mit der Temperatur der Dotierungslösung gesteuert wird.

15. Katalysatoren nach den Ansprüchen 1 oder 3, bei denen die Ablagerung von Re mit dem pH-Wert und der Temperatur der Dotierungslösung gesteuert wird.

16. Verwendung des Raney-Metallfestbettkatalysators nach die Anspruch 1 oder 3 für die Hydrierung gesättigter oder ungesättigter Ester in ihre entsprechenden gesättigten Mono- oder Mehrfachhydroxyalkohole.

17. Verwendung des Raney-Metallfestbettkatalysators nach den Ansprüchen 1 oder 3 für die Teilhydrierung gesättigter oder ungesättigter Ester in ihre entsprechenden Mono- oder Mehrfachhydroxyalkohole.

18. Verwendung des Raney-Metallfestbettkatalysators nach den Ansprüchen 1 oder 3 für die Hydrierung von Maleinsäureanhydrid in Gamma-Butyrolacton, Tetrahydrofuran und 1,4-Butandiol.

19. Verwendung des Raney-Metallfestbettkatalysators nach den Ansprüchen 1 oder 3 für die Hydrierung von Fettsäureester in Fettalkohole.

20. Verwendung des Raney-Metallfestbettkatalysators nach den Ansprüchen 1 oder 3 für die Hydrierung von C-12 bis C-20 Fettsäureester in Fettalkohole.

21. Verwendung des Raney-Metallfestbettkatalysators nach den Ansprüchen 1 oder 3 für die Hydrierung von C-16 bis C-18 Fettsäureester in Fettalkohole.

22. Verwendung des Raney-Metallfestbettkatalysators nach den Ansprüchen 1 oder 3 für die Hydrierung von Maleinsäure in Gamma-Butyrolacton, Tetrahydrofuran und 1,4-Butandiol.

## Claims

1. Shaped, activated Raney metal fixed-bed catalyst, obtainable by a method which essentially comprises the following steps: preparing a mixture of powders consisting essentially of at least one catalyst alloy and optionally at least one binder, and a moistening agent, and optionally an additive selected from the group consisting of a shaping aid, lubricant, plasticizer, pore-producer, and mixtures thereof, wherein said catalyst alloy consists essentially of at least one Raney process metal as catalytically active catalyst metal, a leachable alloy component and optionally a promoter, wherein said binder consists essentially of at least one pure Raney process metal, homogenizing said mixture, shaping said mixture to give a moulded catalyst precursor which still is not activated, calcining said moulded catalyst precursor at a temperature below 850°C to obtain a sintered catalyst precursor, and activating said sintered catalyst precursor by leaching said leachable alloy component with alkali until the leached and thereby activated outer layer has an adjustable thickness of 0.05 to 1 mm, and subsequently washing the final catalyst; doping said catalyst with rhenium as a promoter after said activating by introducing said catalyst into a perrhenic acid solution or solutions of other Re salts, for a sufficient period of time to fix all of the rhenium onto the catalyst or by addition of the rhenium to the unactivated alloy or optionally by addition of the rhenium to the binder.

2. Process for preparing the shaped Raney metal fixed-bed catalyst according to claim 1, said process essentially comprising the following steps: preparing a mixture of powders consisting essentially of at least one catalyst alloy and optionally at least one binder, and a moistening agent, and optionally an additive selected from the group consisting of a shaping aid, lubricant, plasticizer, pore-producer, and mixtures thereof, wherein said catalyst alloy consists essentially of at least one Raney process metal as catalytically active component, a leachable alloy component and optionally a promoter, wherein said binder consists essentially of at least one Raney process metal, homogenizing said mixture, shaping said mixture to give a moulded catalyst precursor which still is not activated, calcining said moulded catalyst precursor at a temperature below 850°C to obtain a sintered catalyst precursor, and activating said sintered catalyst precursor by leaching said leachable alloy component with alkali until the leached and thereby activated outer layer has an adjustable thickness of 0.05 to 1 mm; optionally subsequently washing the final catalyst; **characterized in that** the catalyst is doped with rhenium as a promoter after said activating by introducing said catalyst into a perrhenic acid solution whose pH was initially adjusted for a sufficient period of time to fix all of the rhenium on the catalyst or [sic] the rhenium to the unactivated alloy or optionally by addition of the rhenium to the binder.

3. Catalysts according to claim 1 in which a binder is not required for a stable formed body and the resulting catalyst is doped with Re by introducing the activated catalyst into a perrhenic acid solution for a sufficient amount of time.

4. Catalysts according to claims 1 or 3 in which the Re content is from 0.01% Re to 30% Re.

5. Catalysts according to claims 1 or 3 in which the Re content is from 0.01% Re to 10% Re.

6. Catalysts according to claims 1 or 3 in which the Raney metal is Ni.

7. Catalysts according to claims 1 or 3 in which the Raney metal is Cu.

8. Catalysts according to claims 1 or 3 in which the Raney metal is additionally doped with 0.01-5% chromium in combination with 0.01-5% iron either before or after the doping with Re.

9. Catalysts according to claim 8 in which the Raney metal is nickel and it is additionally doped with 0.01-5% chromium in combination with 0.01-5% iron either before or after the doping with Re.

10. Catalysts according to claims 1 or 3 in which the Raney metal is additionally doped with 0.01-5% chromium before or after the doping with Re.

11. Catalysts according to claims 1 or 3 in which the Raney metal is additionally doped with 0.01-5% iron either before or after the doping with Re.

12. Catalysts according to claims 1 or 3 in which the Raney metal is additionally doped with other promoters such as Ti, Zr, V, Ta, Mo, W, Ru, Pd, Pt, Cu, Zn, or Co either before or after the Re promotion.

13. Catalysts according to claims 1 or 3 in which the deposition of Re is controlled by the pH of the doping solution.

14. Catalysts according to claims 1 or 3 in which the deposition of Re is controlled by the temperature of the doping solution.

15. Catalysts according to claims 1 or 3 in which the deposition of Re is controlled by both the pH and the temperature of the doping solution.

16. Use of the Raney metal fixed-bed catalyst according to claims 1 or 3 for the hydrogenation of saturated or unsaturated esters to their corresponding mono or multiple hydroxy saturated alcohols.

17. Use of the Raney metal fixed-bed catalyst according to claims 1 or 3 for the partial hydrogenation of saturated or unsaturated esters to their corresponding mono or multiple hydroxy alcohols.

18. Use of the Raney metal fixed-bed catalyst according to claims 1 or 3 for the hydrogenation of maleic anhydride to gamma-butyrolactone, tetrahydrofuran, and 1,4-butanediol

19. Use of the Raney metal fixed-bed catalyst according to claims 1 or 3 for the hydrogenation of fatty acid esters to fatty alcohols.

20. Use of the Raney metal fixed-bed catalyst according to claims 1 or 3 for the hydrogenation of C-12 to C-20 fatty acid esters to fatty alcohols.

21. Use of the Raney metal fixed-bed catalyst according to claims 1 or 3 for the hydrogenation of C-16 to C-18 fatty acid esters to fatty alcohols.

22. Use of the Raney metal fixed-bed catalyst according to claims 1 or 3 for the hydrogenation of maleic acid to gamma-butyrolactone, tetrahydrofuran, and 1,4-butanediol.

## Revendications

1. Catalyseur en lit fixe à base de métal de Raney, activé, moulé, accessible par un procédé qui comprend essentiellement les étapes suivantes :
- préparation d'un mélange à base de poudres qui consistent pour l'essentiel en au moins un alliage de catalyseur et éventuellement au moins un agent liant et en un agent mouillant et en cas de besoin d'un additif choisi dans le groupe qui consiste en un adjuvant de modelage, un agent lubrifiant, un agent de ramollissement, un agent porogène et de mélanges de ceux-ci, dans lequel ledit alliage de catalyseur consiste pour l'essentiel en au moins un métal de processus de Raney en tant que métal de catalyseur actif catalytiquement, en un composant d'alliage lessivable, et en cas de besoin un promoteur dans lequel ledit agent liant consiste pour l'essentiel en un métal de processus de Raney pur,
- homogénéisation dudit mélange,
- moulage dudit mélange pour obtenir un précurseur de catalyseur moulé qui n'est toujours pas activé,
- calcination dudit précurseur de catalyseur moulé à une température inférieure à 850°C pour produire un précurseur de catalyseur fritté et activation dudit précurseur de catalyseur fritté par lessivage dudit composant d'alliage lessivable, avec un alcali jusqu'à ce que la couche externe lessivée et activée de cette façon, présente une épaisseur réglable comprise entre 0,05 et 1 mm et ensuite lavage du catalyseur terminé,
- dopage dudit catalyseur avec du rhénium en tant que promoteur, après ladite activation par introduction dudit catalyseur dans une solution d'acide perrhénique ou des solutions à base d'autres sels de rhénium pendant un laps de temps suffisant pour fixer la totalité du rhénium sur le catalyseur ou par addition du rhénium dans l'alliage inactivé ou le cas échéant par addition de rhénium dans l'agent liant.

2. Procédé de préparation du catalyseur en lit fixe à base de métal de Raney selon la revendication 1,
dans lequel ledit procédé comprend pour l'essentiel les étapes suivantes :
- préparation d'un mélange à base de poudres qui consistent pour l'essentiel en au moins un alliage de catalyseur et le cas échéant au moins un agent liant, et en un agent de mouillage et en cas de besoin un additif choisi dans le groupe qui consiste en un agent adjuvant de modelage, un agent de ramollissement, un agent porogène et des mélanges de ceux-ci, dans lequel ledit alliage de catalyseur consiste pour l'essentiel en au moins un métal de processus de Raney comme composant actif catalytiquement, un composant d'alliage lessivable, et en cas de besoin un promoteur, dans lequel ledit agent liant consiste pour l'essentiel en au moins un métal de processus de Ranay,
- homogénéisation dudit mélange,
- moulage dudit mélange pour obtenir un précurseur de catalyseur moulé qui n'est pas encore activé,
- calcination dudit précurseur de catalyseur moulé, à une température inférieure à 850°C, afin de produire un précurseur de catalyseur fritté, et activation dudit précurseur de catalyseur fritté par lessivage dudit composant d'alliage lessivable avec un alcali, jusqu'à ce que la couche externe lessivée et de cette façon activée, possède une épaisseur réglable comprise entre 0,05 et 1 mm,
- ensuite en cas de besoin lavage du catalyseur terminé,
**caractérisé en ce que**
le catalyseur est dopé avec du rhénium comme promoteur après ladite activation, procédé dans lequel ledit catalyseur est introduit dans une solution perrhénique dont la valeur de pH a été ajustée pendant un laps de temps suffisant pour fixer la totalité du rhénium sur le catalyseur ou introduire le rhénium dans l'alliage non activé ou le cas échéant par addition de rhénium dans l'agent liant.

3. Catalyseurs selon la revendication 1
dans lesquels
aucun agent liant pour un solide moulé stable n'est nécessaire et dans lesquels le catalyseur résultant est dopé avec du rhénium, dans lesquels le catalyseur activé est introduit dans une solution d'acide perrhénique pendant un laps de temps suffisant.

4. Catalyseurs selon la revendication 1 ou 3,
dans lequel
la teneur en Re se situe entre 0,01 de Re et 30 % de Re.

5. Catalyseurs selon la revendication 1 ou 3,
dans lesquels
la teneur en Re se situe entre 0,01 % de Re et 10 % de Re.

6. Catalyseurs selon la revendication 1 ou 3,
dans lesquels
le métal de Raney est du nickel.

7. Catalyseurs selon la revendication 1 ou 3,
dans lesquels
le métal de Raney est du cuivre.

8. Catalyseurs selon la revendication 1 ou 3,
dans lesquels
le métal de Raney est dopé en supplément avec de 0,01 à 5 % de chrome en combinaison avec de 0,01 à 5 % de fer, avant ou après le dopage par le Re.

9. Catalyseurs selon la revendication 8,
dans lesquels
le métal de Raney est le nickel et en supplément est dopé avec de 0,01 à 5 % de chrome en combinaison avec de 0,01 à 5 % de fer avant ou après le dopage par le Re.

10. Catalyseurs selon la revendication 1 ou 3,
dans lesquels
le métal de Raney est dopé en supplément avec de 0,01 à 5 % de chrome, avant ou après le dopage par le Re.

11. Catalyseurs selon la revendication 1 ou 3,
dans lesquels
le métal de Raney est dopé en supplément avec de 0,01 à 5 % de fer, avant ou après le dopage par le Re.

12. Catalyseurs selon la revendication 1 ou 3,
dans lesquels
le métal de Raney est dopé en supplément avec d'autres promoteur comme Ti, Zr, V, Ta, Mo, W, Ru, Pd, Pt, Cu, Zn ou Co, avant ou après le dopage par Re.

13. Catalyseurs selon la revendication 1 ou 3,
dans lesquels
la sédimentation du Re est contrôlée avec la valeur du pH de la solution de dopage.

14. Catalyseurs selon la revendication 1 ou 3,
dans lesquels
la sédimentation du Re est contrôlée avec la température de la solution de dopage.

15. Catalyseurs selon la revendication 1 ou 3,
dans lesquels
la sédimentation de Re est contrôlée avec la valeur du pH et la température de la solution de dopage.

16. Utilisation du catalyseur en lit fixe à base de métal de Raney selon la revendication 1 ou 3, en vue de l'hydrogénation d'esters saturés ou non saturés en leurs alcools saturés correspondants mono ou plusieurs fois hydroxylés.

17. Utilisation du catalyseur en lit fixe à base de métal de Raney, selon la revendication 1 ou 3, pour l'hydrogénation partielle d'esters saturés ou non saturés en alcools correspondants mono ou plusieurs fois hydroxylés.

18. Utilisation du catalyseur en lit fixe à base de métal de Raney selon la revendication 1 ou 3, pour l'hydrogénation d'anhydride maléique en gamma-butyrolactone, tétrahydrofurane et 1,4-butanediol.

19. Utilisation du catalyseur en lit fixe à base de métal de Raney selon la revendication 1 ou 3, pour l'hydrogénation d'esters d'acides gras en alcools gras.

20. Utilisation du catalyseur en lit fixe à base de métal de Raney selon la revendication 1 ou 3, pour l'hydrogénation d'esters d'acides gras en C₁₂ à C₂₀ en alcools gras.

21. Utilisation du catalyseur en lit fixe à base de métal de Raney selon la revendication 1 ou 3, pour l'hydrogénation d'esters d'acides gras en C₁₃ à C₁₈ en alcools gras.

22. Utilisation du catalyseur en lit fixe à base de métal de Raney selon la revendication 1 ou 3, pour l'hydrogénation d'acide maléique en gamma butyrolactone, tétrahydrofurane et 1,4-butanediol.
